# EUROPEAN PATENT APPLICATION

(11) **EP 4 501 304 A1**
(43) Date of publication of application: **05.02.2025**
(21) Application number: 23780777.1
(22) Date of filing: 29.03.2023
(51) Int. Cl.: A61K 6/30, A61K 6/15, A61K 6/60

(54) **ADHESIVE COMPOSITION FOR DENTAL USE**

(30) Priority: 31.03.2022 JP 2022061107
(71) Applicant: GC Corporation, Sunto-gun, Shizuoka 410-1307 (JP)
(72) Inventor: OHARA, Yuki, Tokyo 174-8585 (JP); TAKAHASHI, Makoto, Tokyo 174-8585 (JP); KASAI, Yuki, Tokyo 174-8585 (JP)
(74) Representative: Müller-Boré & Partner Patentanwälte PartG mbB
(86) International application number: PCT/JP2023/012995
(87) International publication number: WO 2023/190769

(57) **Abstract**

A dental adhesive composition includes a polymer including at least a constituent unit represented by a formula (1), and having a weight-average molecular weight (Mw) of 2,000 or greater and 55,000 or less, 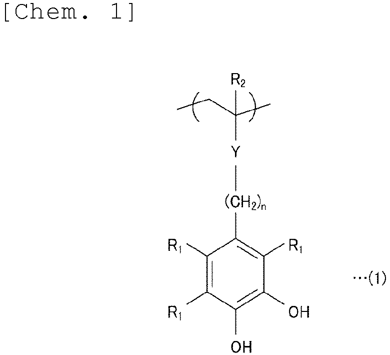 [in the formula (1), Y is an amide bond or an ester bond, n is an integer of 1 or greater and 10 or less, multiple R₁ are each independently a hydrogen atom, a hydroxyl group, or an alkyl group having a carbon number of 1 or greater and 6 or less, and R₂ is a hydrogen atom or an alkyl group having a carbon number of 1 or greater and 6 or less].

## Description

### TECHNICAL FIELD

The present invention relates to dental adhesive compositions.

### BACKGROUND ART

Use of phosphoric acid ester-based monomers as a component of dental adhesive compositions has been known. Among phosphoric acid ester-based monomers, 10-methacryloyloxydecyl dihydrogen phosphate (MDP) is widely used because of desired adhesion to dental tissues (see, for example, Patent Document 1).

### CITATION LIST

### PATENT DOCUMENT

Patent Document 1: Japanese Unexamined Patent Application Publication No. H03-50724

### SUMMARY OF THE INVENTION

### Technical Problem

However, a composition including a phosphoric acid ester-based monomer may cause irritation on tooth surfaces or in oral cavities due to a phosphoric acid group included in the monomer. Therefore, there is a need for a dental adhesive composition that is unlikely to cause irritation.

One aspect of the present invention aims to provide a dental adhesive composition that is unlikely to cause irritation on tooth surfaces or oral cavities.

### SOLUTION TO THE PROBLEM

One aspect of the present invention is a dental adhesive composition that includes a polymer including at least a constituent unit represented by a formula (1) and has a weight-average molecular weight (Mw) of 2,000 or greater and 55,000 or less. 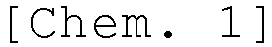 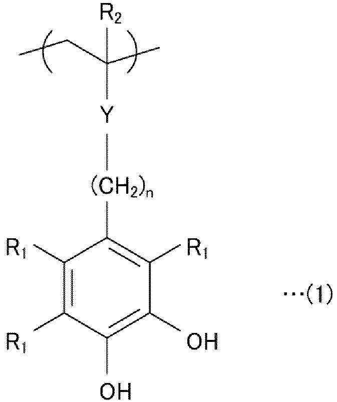 [In the formula (1), Y is an amide bond or an ester bond, n is an integer of 1 or greater and 10 or less, multiple R₁ are each independently a hydrogen atom, a hydroxyl group, or an alkyl group having a carbon number of 1 or greater and 6 or less, and R₂ is a hydrogen atom or an alkyl group having a carbon number of 1 or greater and 6 or less.]

### EFFECTS OF THE INVENTION

According to one aspect of the present invention, a dental adhesive composition that is unlikely to cause irritation on tooth surfaces or oral cavities can be provided.

### DETAILED DESCRIPTION OF THE EMBODIMENTS

The dental adhesive according to one embodiment of the present invention includes a polymer including a constituent unit (repeating unit) represented by a general formula (1). 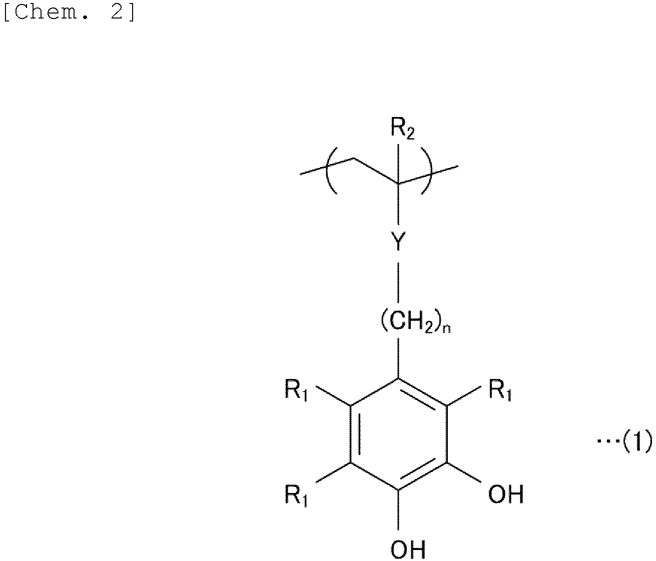 [In the formula (1), Y is an amide bond (-NH-CO-) or an ester bond (-COO-), n is an integer of 1 or greater and 10 or less, multiple R₁ are each independently a hydrogen atom, a hydroxyl group, or an alkyl group having a carbon number of 1 or greater and 6 or less, and R₂ is a hydrogen atom or an alkyl group having a carbon number of 1 or greater and 6 or less], and having a weight-average molecular weight of 2,000 or greater and 55,000 or less. The composition that includes the polymer including the constituent unit represented by the general formula (1) and having the predetermined weight-average molecular weight has an excellent bond strength to dental tissues, particularly to enamel, dentin, or both enamel and dentin. Moreover, pH of the dental adhesive composition is biocompatible, thus it is unlikely that irritation is caused when the dental adhesive composition is used in an oral cavity, and high storage stability of the dental adhesive composition is also achieved.

Y in the general formula (1) is preferably an amide bond; n is preferably an integer of 1 or greater and 5 or less, and more preferably an integer of 1 or 2; among the multiple R₁, two or more R₁ are preferably each a hydrogen atom, and more preferably, all three R₁ are hydrogen atoms, i.e., a side chain of the polymer including a catechol group (a group in which a hydrogen atom in an unsubstituted position of catechol is removed); and R₂ is preferably a hydrogen atom or a methyl group.

In the case where Y in the general formula (1) is an amide bond, the constituent unit represented by the general formula (1) is a constituent unit derived from (meth)acrylamide. In the present specification, the term "(meth)acryl" encompasses acryl, methacryl, or both acryl and methacryl. Moreover, the term "(meth)acrylate" encompasses acrylate, methacrylate, or both acrylate and methacrylate, and the term "(meth)acryloyloxy" encompasses methacryloyloxy, acryloyloxy, or both methacryloyloxy and acryloyloxy. Therefore, the general formula (1) includes a homopolymer composed of a constituent unit derived from acrylamide, a homopolymer composed of a constituent unit derived from methacrylamide, a copolymer including a constituent unit derived from acrylamide and a constituent unit derived from methacrylamide, and a blend of a homopolymer composed of a constituent unit derived from acrylamide and a homopolymer composed of a constituent unit derived from methacrylamide.

In the case where Y is an amide bond in the general formula (1), a constituent unit of the general formula (1) is preferably a constituent unit derived from N-(3,4-dihydroxyphenylmethyl) (meth)acrylamide, N-[2-(3,4-dihydroxyphenyl)ethyl] (meth)acrylamide (dopamine (meth)acrylamide), or N-[3-(3,4-dihydroxyphenyl)propyl](meth)acrylamide, N-[4-(3,4-dihydroxyphenyl)butyl](meth)acrylamide, or the like in view of improvement in a bond strength of a resultant dental adhesive composition.

Among the above constituent units, a constituent unit derived from N-(3,4-dihydroxyphenylmethyl)acrylamide represented by the following formula (1a) and a constituent unit derived from N-[2-(3,4-dihydroxyphenyl)ethyl]acrylamide(dopamine acrylamide) represented by the following formula (1b) are preferred.

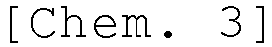

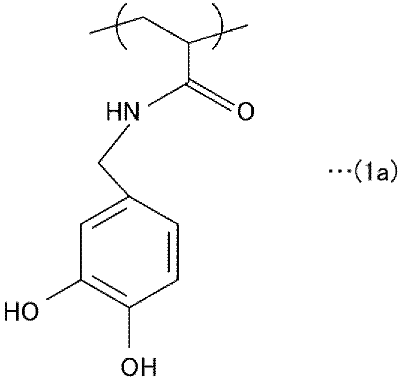

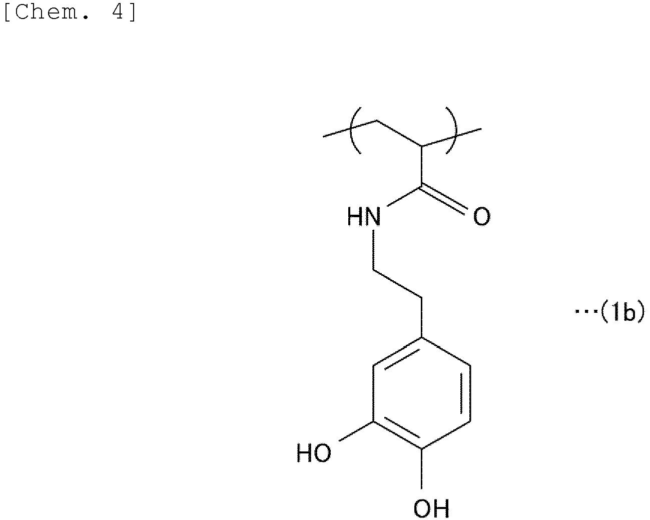

The polymer including the constituent represented by the above general formula (1) may be a homopolymer, or a copolymer further including a constituent unit other than the constituent unit represented by the general formula (1). In the case where the polymer of the present embodiment is a copolymer, the polymer may be a copolymer including a constituent unit represented by the general formula (1) and a constituent unit represented by the following general formula (2): 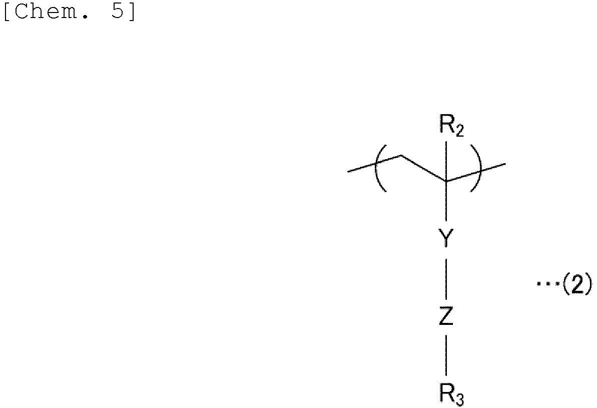 [In the formula (2), Y is an amide bond or an ester bond, Z is an alkylene group having a carbon number of 1 or greater and 10 or less or an ethylene glycol (oxyethylene group) having 1 or more and 5 or less repeating units, multiple R₂ are each independently a hydrogen atom or an alkyl group having a carbon number of 1 or greater and 6 or less, and R₃ is a hydrogen atom, a methyl group, or an amino group]. In the case where the polymer of the present embodiment is the above copolymer, the polymer is easily dissolved in a solvent, such as water, acetone, or the like, and thus operability can be improved in an application of a dental adhesive composition including a solvent or an application of a dental adhesive composition that is mixed with a solvent when used.

In the case where the polymer of the present embodiment is a copolymer, the polymer is preferably a random copolymer. Moreover, the polymer is more preferably a random copolymer including a constituent unit represented by the general formula (1) and a constituent unit represented by the general formula (2).

In the general formula (2), Y is preferably an amide bond. Z may be preferably an alkylene group having a carbon number of 1 or greater and 10 or less or ethylene glycol including 1 or more and 5 or less repeating units, and more preferably an alkylene group having a carbon number of 1 or greater and 5 or less. Moreover, R₃ is preferably an amino group. Furthermore, R₂ is preferably a hydrogen atom or a methyl group.

In the case where Y in the general formula (2) is an amide bond, the constituent unit is preferably a constituent unit derived from N-(2-aminoethyl) (meth)acrylamide, a constituent unit derived from N-(3-aminopropyl) (meth)acrylamide, a constituent unit derived from N-(4-aminobutyl) (meth)acrylamide, a constituent unit derived from N-(5-aminopentyl) (meth)acrylamide, or the like is preferred in view of improvement in a bond strength of a resultant dental adhesive composition. Among the above constituent units, one or more constituents unit selected from a constituent unit derived from N-(3-aminopropyl)acrylamide and a constituent unit derived from N-(4-aminobutyl)acrylamide are more preferred.

In the case where the polymer of the present embodiment is a random copolymer including a constituent unit represented by the general formula (1) and a constituent unit represented by the general formula (2), the polymer can be represented by the following general formula (3): 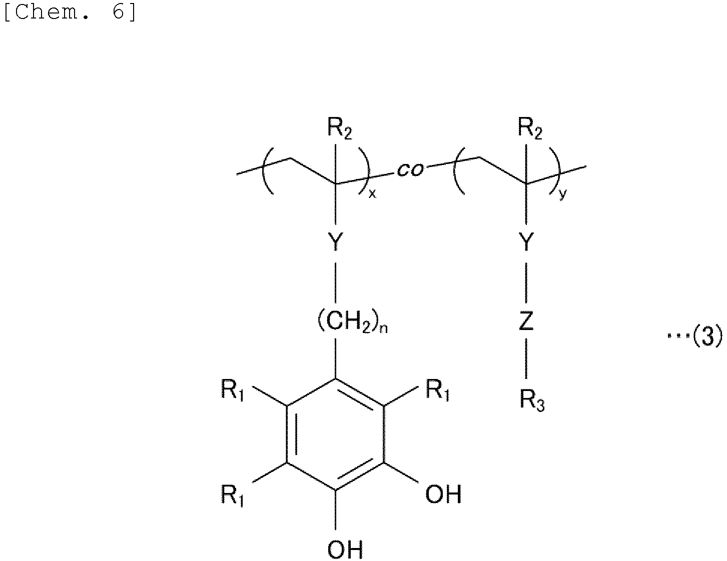 [In the formula (3), multiple Y are each independently an amide bond or an ester bond, Z is an alkylene group having a carbon number of 1 or greater and 10 or less or ethylene glycol including 1 or more and 5 or less repeating units, n is an integer of 1 or greater and 10 or less, multiple R₁ are each independently a hydrogen atom, a hydroxyl group, or an alkyl group having a carbon number of 1 or greater and 6 or less, multiple R₂ are each independently a hydrogen atom or an alkyl group having a carbon number of 1 or greater and 6 or less, and R₃ is a hydrogen atom, a methyl group, or an amino group]. Note that, "-co-" in the general formula (3) indicates that the constituent units linked with the "-co-" are randomly arranged.

In the general formula (3), all Y are preferably amide bonds. Moreover, Z may be an alkylene group having a carbon number of 1 or greater and 10 or less or ethylene glycol including 1 or more and 5 or less repeating units, and more preferably an alkylene group having a carbon number of 1 or greater and 5 or less. R₂ is preferably a hydrogen atom or a methyl group. R₃ is preferably an amino group.

In the general formula (3), x/(x + y) is preferably 0.1 or greater and less than 1, and may be 0.15 or greater and 0.8 or less, or 0.2 or greater and 0.5 or less. By setting the value of x/(x+y) within the above ranges, a bond strength of the dental adhesive composition to dental tissues, particularly to dentin, can be improved, and dispersibility of the polymer in the dental adhesive composition can be improved.

In the case where all Y in the general formula (3) are amide bonds, specific examples of the polymer of the present embodiment include polymers represented by the following formulae (3a) and (3b):

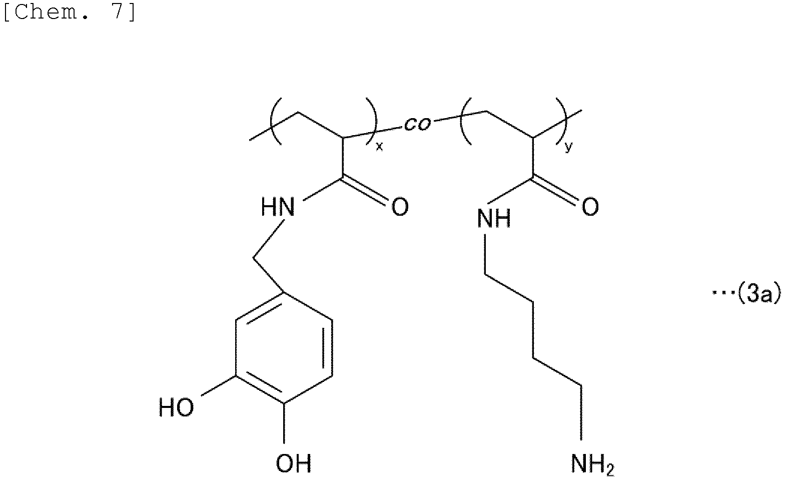

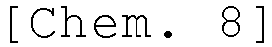

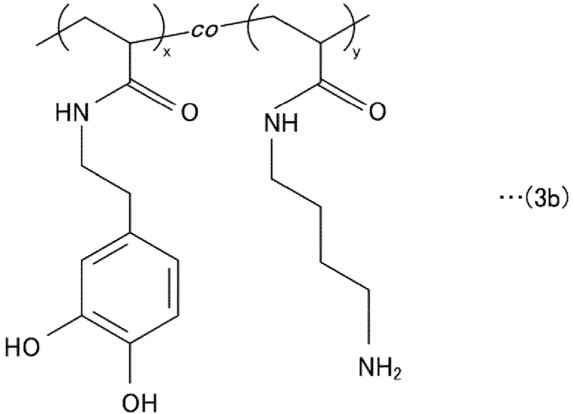

The polymers represented by the above formulae (3a) and (3b) are preferred in view of improvement in a bond strength of the dental adhesive composition to dental tissues.

The polymer terminal in the present embodiment is preferably a hydrogen atom, whether the polymer is a homopolymer or copolymer.

The above polymer has a weight-average molecular weight (Mw) of 2,000 or greater and 55,000 or less, preferably 3,000 or greater and 40,000 or less, more preferably 5,000 or greater and 30,000 or less, and yet more preferably 5,000 or greater and 15,000 or less. Since the polymer has the weight-average molecular weight of the above ranges, the polymer can be suitably blended with other components. Therefore, a process for preparing a dental adhesive composition can be readily carried out, operability can be improved during use of the dental adhesive composition, and a suitable bond strength of the dental adhesive composition can be ensured.

The weight-average molecular weight (Mw) of the polymer may be directly measured, or an estimated value may be used. For example, when a polymer precursor (polymer before side chain formation) is prepared from a starting monomer during a process of synthesis of the polymer, a degree of polymerization is calculated from a molar ratio of the starting monomer added to the chain transfer agent added (Mₘₒ/M_{ct}, where M_{ct} is a mole number of the chain transfer agent and Mₘₒ is a mole number of the starting monomer) and a reaction rate (polymerization rate) of a polymerization reaction, and a weight-average molecular weight may be calculated further considering an introduction rate of a side chain functional group.

Moreover, a value of a ratio (Mw/Mn) of the weight average molecular weight to a number-average molecular weight may be preferably 1 or greater and 2 or less, more preferably 1 or greater and 1.7 or less, and yet more preferably 1 or greater and 1.5 or less. Since the Mw/Mn is in the above ranges, a narrower molecular weight distribution of the polymer can be achieved so that dispersibility of the polymer in the dental adhesive composition can be further improved. Therefore, effects, such as easiness of a process for preparing a dental adhesive composition, improvement in operability during use of the dental adhesive composition, improvement in a suitable bond strength of the dental adhesion composition, and the like, can be further enhanced.

As pH of the dental adhesive composition in a solution, for example, pH measured on a solution obtained by dissolving 1 g of the dental adhesive composition in 1 mL of an aqueous solvent may be preferably 2 or higher and 10 or lower, and more preferably 3 or higher and 9 or lower. Therefore, the dental adhesive composition of the present embodiment does not give irritation due to acidity or basicity, excels in usability, and can stably maintain properties thereof against fluctuations of environmental conditions even when stored over a long period.

The dental adhesive composition of the present embodiment may include a polymerizable monomer in addition to the above polymer. The polymerizable monomer is preferably (meth)acrylate. As the (meth)acrylate is included in the dental adhesive composition, a bond strength of the dental adhesive composition to dental tissues can be improved.

An amount of the polymerizable monomer in the dental adhesive composition may depend on use of the dental adhesive composition. The amount of the polymerizable monomer in the dental adhesive composition is preferably from 1% by mass to 85% by mass, and more preferably from 5% by mass to 75% by mass relative to a total amount of the dental adhesive composition.

The (meth)acrylate is preferably (meth)acrylate including no acid group. Specific examples of the (meth)acrylate including no acid group include: methyl(meth)acrylate, ethyl (meth)acrylate, isopropyl (meth)acrylate, n-butyl (meth)acrylate, isobutyl (meth)acrylate, hydroxypropyl (meth)acrylate, tetrahydrofurfuryl (meth)acrylate, glycidyl(meth) acrylate, 2-hydroxyethyl (meth)acrylate, 2-hydroxypropyl(meth)acrylate, 3-hydroxypropyl(meth)acrylate, 2-methoxyethyl(meth) acrylate, 2-ethoxyethyl(meth)acrylate, 2-methylhexyl(meth)acrylate, 2-ethylhexyl(meth)acrylate, benzyl(meth)acrylate, 2-hydroxy-1,3-di(meth)acryloyloxypropane, ethylene glycol di(meth)acrylate, diethylene glycol di(meth)acrylate, triethylene glycol di(meth)acrylate, butylene glycol di(meth)acrylate, neopentyl glycol di(meth)acrylate, 1,3-butanediol di(meth)acrylate, 1,4-butanediol di(meth)acrylate, 1,6-hexanediol di(meth)acrylate, trimethylolpropane tri(meth)acrylate, trimethylolethane tri(meth)acrylate, pentaerythritol tri(meth)acrylate, trimethylolmethane tri(meth)acrylate, pentaerythritol tetra(meth)acrylate, polybutylene glycol di(meth)acrylate, bisphenol A diglycidyl (meth)acrylate, di-2-(meth)acryloyloxyethyl-2,2,4-trimethylhexamethylene dicarbamate, 1,3,5-tris[1,3-bis{ (meth)acryloyloxy}-2-propoxycarbonylaminohexane]-1,3,5-(1H, 3H, 5H)triazine-2,4,6-trione, 2,2-bis[4-(3-(meth) acryloyloxy-2-hydroxypropyl)phenyl]propane, N,N'-(2,2,4-trimethylhexamethylene)bis[2-(aminocarboxy)propane-1,3-diol]tetramethacrylate, a (meth)acrylate of a urethane oligomer, which is composed of 2,2'-bis(4-hydroxycyclohexyl)propane, 2-oxepanone, hexamethylene diisocyanate, and 2-hydroxyethyl (meth)acrylate, a (meth)acrylate of a urethane oligomer, which is composed of 1,3-butanediol, hexamethylene diisocyanate, and 2-hydroxyethyl (meth)acrylate, and the like. The above (meth)acrylates may be used alone or in combination. Among the above (meth)acrylates, a combination of 2-hydroxyethyl methacrylate (HEMA), triethylene glycol dimethacrylate (TEGDMA), and bisphenol A glycidyl methacrylate (Bis-GMA) is preferably used.

The dental adhesive composition according to the present embodiment may include a (meth)acrylate including an acid group, such as a phosphoric acid group, a pyrophosphoric acid group, a thiophosphoric acid group, a carboxylic acid group, a sulfonic acid group, a phosphonic acid group, or the like. For example, the dental adhesive composition may include 10-methacryloyloxydecyl dihydrogen phosphate (MDP). In the present embodiment, an amount of MDP is however preferably 10% by mass or less, and more preferably 5% by mass or less relative to a total amount of the dental adhesive composition. Thus, irritation to dental tissues and oral cavities can be minimized during use of the dental adhesive composition, and a product with great usability can be obtained. Moreover, the dental adhesive composition is preferably substantially free from 10-methacryloyloxydecyl dihydrogen phosphate. In the present specification, the phrase "substantially free from" encompasses the meaning that a relevant component is not intentionally formulated, but may be included as unavoidable impurities. In the case where the relevant component is included as unavoidable impurities, an amount thereof is preferably less than 1% by mass, and more preferably less than 0.5% by mass relative to a total amount of the dental adhesive composition.

From a standpoint of further reduction in irritation to dental tissues and oral cavities, an amount of the polymerizable monomer including a phosphoric acid group may be preferably 10% by mass or less, more preferably 5% by mass or less relative to a total amount of the dental adhesive composition, and the dental adhesive composition is more preferably substantially free from the polymerizable monomer including a phosphoric acid group. Moreover, an amount of the polymerizable monomer including an acid group may be preferably 10% by mass or less, more preferably 5% by mass or less relative to a total amount of the dental adhesive composition, and the dental adhesive composition is more preferably substantially free from the (meth)acrylate including an acid group.

Moreover, in the present embodiment, an amount of the above polymer, **i.e.,** the polymer including the constituent unit represented by the general formula (1) and having the weight-average molecular weight (Mw) of 2,000 or greater and 55,000 or less, in the dental adhesive composition may be preferably 5 parts by mass or greater and 300 parts by mass or less, and more preferably 10 parts by mass or greater and 200 parts by mass or less relative to 100 parts by mass of the dental adhesive composition.

Moreover, the dental adhesive composition according to the present embodiment may further include an inorganic filler such as a glass power and the like.

An amount of the organic filler may depend on use of the dental adhesive composition, but may be 1% by mass or greater and 80% by mass or less relative to a total amount of the dental adhesive composition. In the case where the dental adhesive composition according to the present embodiment is a dental composite resin, the amount of the inorganic filler is preferably 40% by mass or greater and 80% by mass or less relative to a total amount of the dental composite resin.

Moreover, the dental adhesive composition according to the present embodiment may be used as a composite resin for tooth filling, a dental primer, a dental adhesive material, a dental coating material, a dental composite resin cement, a dental splinting material, or the like. Moreover, the dental adhesive composition is preferably a photopolymerizable composition that can be polymerized by light.

As a photopolymerization initiator, for example, camphorquinone (CQ), ethyl 4-dimethylaminobenzoate (EPA), diphenyl(2,4,6-trimethylbenzoyl)phosphine oxide (TPO), or the like can be used.

As a polymerization inhibitor, for example, dibutylhydroxytoluene (2,6-di-tert-butyl-p-cresol) (BHT), 6-tert-butyl-2,4-xylenol, or the like can be used.

Moreover, the dental adhesive composition may further include an antioxidant, an anti-tarnish agent, a plasticizer, a surfactant, an antibacterial agent, a UV absorber, a pigment, a dye, fibers, and the like, as long as functions and effects obtained by the present embodiment are not adversely affected.

### Examples

In experiments below, dental adhesive compositions (dental composite resins) having different formulations were prepared, and properties thereof were evaluated.

### [Preparation of composition]

### <Preparation of Component A>

Component A is a substance contributing to adhesion. As Component A, a polymer of any of Structure 1 to Structure 4, or Monomer a (dopamine methacrylamide (DMA), molecular weight of 221) was used (the structure of the monomer a is also presented below).

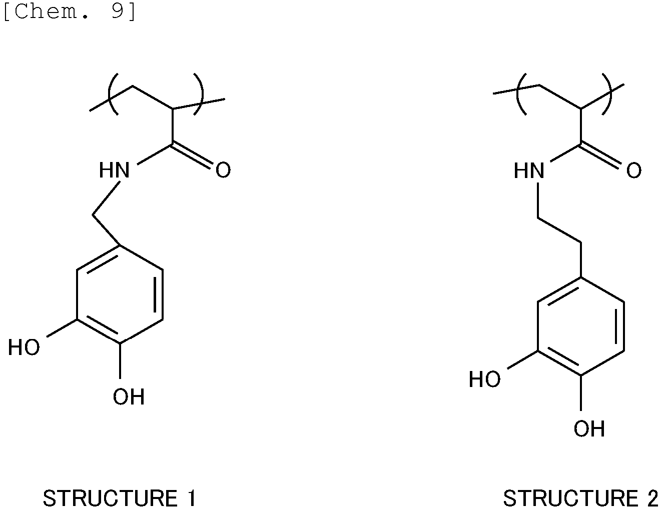

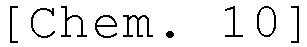

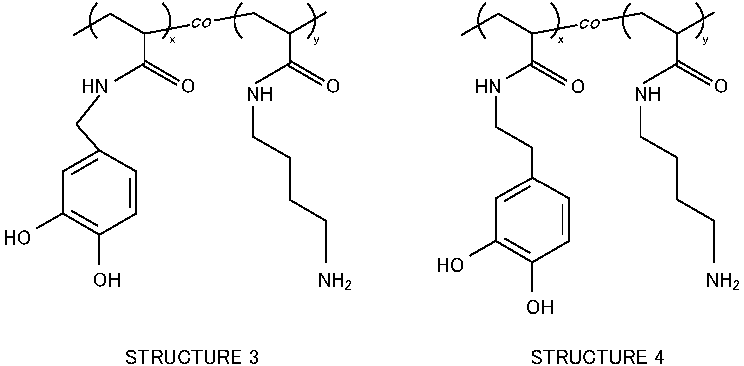

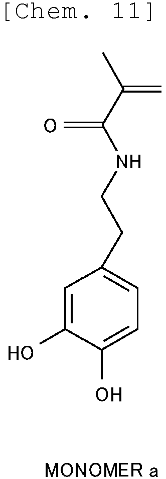

Each of Structure 1 and Structure 2 is a homopolymer composed of the constituent unit represented by the corresponding structural formula as presented. Moreover, each of Structure 3 and Structure 4 is a random copolymer including the constituent units represented by the corresponding structural formula as presented. In Structure 3, x/(x + y) was 0.3 regardless of a molecular weight of the polymer. In Structure 4, x/(x + y) was 0.3 regardless of a molecular weight of the polymer. The structure of the polymer, the weight-average molecular weight (Mw) of the polymer, and the value of the ratio (Mw/Mn) of the weight-average molecular weight to number-average molecular weight of the polymer used in each example are presented in Table 1.

### <Preparation of composition>

In each example, Component A above was blended together with other constituent components to obtain a composition. In each example, components were blended according to any of Formulations 1 to 4 presented in Table 2, except the case where the components could not be blended due to properties, viscosity, etc. of the polymer.

**[Table 1]**

| | EXAMPLE 1 | EXAMPLE 2 | EXAMPLE 3 | EXAMPLE 4 | EXAMPLE 5 | EXAMPLE 6 | EXAMPLE 7 | EXAMPLE 8 | EXAMPLE 9 | COMPARATIVE EXAMPLE 1 | COMPARATIVE EXAMPLE 2 | COMPARATIVE EXAMPLE 3 | COMPARATIVE EXAMPLE 4* | COMPARATIVE EXAMPLE 5* | COMPARATIVE EXAMPLE 6 |
|---|---|---|---|---|---|---|---|---|---|---|---|---|---|---|---|
| COMPONENT A | POLYMER STRUCTURE 4 | POLYMER STRUCTURE 4 | POLYMER STRUCTURE 2 | POLYMER STRUCTURE 4 | POLYMER STRUCTURE 4 | POLYMER STRUCTURE 4 | POLYMER STRUCTURE 4 | POLYMER STRUCTURE 1 | POLYMER STRUCTURE 3 | POLYMER STRUCTURE 2 | MONOMER | MONOMER | POLYMER STRUCTURE 2 | POLYMER STRUCTURE 2 | - |
| WEIGHT-AVERAGE MOLECULAR WEIGHT OF POLYMER (Mw) | 53000 | 30300 | 10400 | 10600 | 10600 | 7500 | 7500 | 2900 | 2200 | 80400 | - | - | 207000 | 144900 | - |
| Mw/Mn OF POLYMER | 1.7 | 1.5 | 1.5 | 1.5 | 1.5 | 1.3 | 1.3 | 1.3 | 1.3 | 1.7 | - | - | 10.1 | 1.7 | - |
| MOLAR RATIO OF START NG MONOMER 0 CHAIN TRANSFER AGENT | 350 | 200 | 200 | 70 | 70 | 50 | 50 | 10 | 15 | 530 | - | - | 1000 | 700 | - |
| FORMULATION OF COMPOSITION | FORMULATION 2 | FORMULATION 2 | FORMULATION 2 | FORMULATION 1 | FORMULATION 2 | FORMULATION 2 | FORMULATION 3 | FORMULATION 2 | FORMULATION 2 | FORMULATION 2 | FORMULATION 1 | FORMULATION 2 | NOT BLENDED | NOT BLENDED | FORMULATION 4 |
| VISCOSITY OF UNDILUTED SOLUTION [mPa▪s/25°C] | 3410 | 1290 | 920 | 740 | 740 | 530 | 530 | 450 | 420 | 7450 | 310 | 310 | NOT MEASURED | NOT MEASURED | 300 |
| CONSISTENCY OF PASTE [mm] | B | B | A | A | A | A | A | A | A | C | A | A | NOT MEASURED | NOT MEASURED | A |
| BOND STRENGTH TO ENAMEL [MPa] | B | A | A | A | A | A | A | A | A | B | C | C | NOT MEASURED | NOT MEASURED | A |
| BOND STRENGTH TO DENTIN [MPa] | B | B | B | A | A | A | A | B | B | C | C | C | NOT MEASURED | NOT MEASURED | A |
| IRRITATION | A | A | A | B | A | A | A | A | A | A | B | B | NOT MEASURED | NOT MEASURED | C |

| | | | | | | | | | | | | | | | |
|---|---|---|---|---|---|---|---|---|---|---|---|---|---|---|---|
| Comparative Example 4* and Comparative Example 5*: Due to excessively high viscosity of the polymer, the polymer could not be blended with other components, thus measurement and evaluation could not be performed | | | | | | | | | | | | | | | |

**[Table 2]**

| | FORMULATION 1 | FORMULATION 2 | FORMULATION 3 | FORMULATION 4 |
|---|---|---|---|---|
| COMPONENT A | 5 | 8 | 20 | 0 |
| MDP | 3 | 0 | 0 | 8 |
| HEMA | 5 | 5 | 12.5 | 5 |
| TEGDMA | 10 | 10 | 25 | 10 |
| Bis-GMA | 14 | 14 | 35 | 14 |
| CQ | 1 | 1 | 2.5 | 1 |
| EPA | 1.9 | 1.9 | 4.75 | 1.9 |
| BHT | 0.1 | 0.1 | 0.25 | 0.1 |
| GLASS FILLER | 60 | 60 | 0 | 60 |
| TOTAL | 100 | 100 | 100 | 100 |

| | | | | |
|---|---|---|---|---|
| ***Values** in Table are based on % by mass | | | | |

The abbreviations in Table 2 are as follows:
MDP: 10-methacryloyloxydecyl dihydrogen phosphate
HEMA: 2-hydroxyethylmethacrylate
TEGDMA: triethylene glycol dimethacrylate
Bis-GMA: bisphenol A glycidyl methacrylate (2,2-bis[4-(2-hydroxy-3-methacyloyloxypropoxy)phenyl]propane)
CQ: camphorquinone
EPA: ethyl 4-dimethylaminobenzoate
BHT: dibutylhydroxytoluene

### [Measurement and Evaluation]

### <Weight-average molecular weight (Mw) of polymer>

A weight-average molecular weight (Mw) of the polymer was determined from a molar ratio of a starting monomer added relative to a chain transfer agent, a reaction rate (polymerization rate), and a rate (side chain introduction rate) of a side chain introduced into a polymer precursor, to which side chains had not been yet added, in the polymerization reaction for synthesizing polymer.

The method for determining the weight-average molecular weight will be specifically explained through a production method of a polymer of Structure 4 used in Example 1. In order to obtain a polymer of Structure 4 in Example 1, first, pentafluorophenyl acrylate (PFA) serving as a starting monomer was allowed to react with dibenzyl dithiobenzoate serving as a chain transfer agent to undergo a polymerization reaction at a molar ratio of an amount of the chain transfer agent added to an amount of PFA added being 1:350 as presented in Reaction Scheme **I,** thereby yielding a polymer of pentafluorophenyl acrylate (pPFA). Since peaks derived from a vinyl group were not observed by NMR (specifically, the progress of the polymerization reaction was assumed to be 100%), the degree of the polymerization (chain length) was estimated to be 350. Thereafter, pPFA, dopamine hydrochloride, and 1,4-diaminobutane were allowed to react at a molar ratio of an amount of pPFA to an amount of the dopamine hydrochloride to an amount of the **1,4-**diaminobutane being **1:0.3:0.7** as presented in Reaction Scheme II, thereby producing a polymer of Structure 4 used a Component A (side chain reaction). Based on the estimated degree of the polymerization being 350, the molar ratio of the dopamine hydrochloride amount to the 1,4-diaminobutane amount being 0.3:0.7, the molecular weight of the dopamine acrylamide being 207, and the molecular weight of the aminobutane acrylamide being 128, the weight-average molecular weight (Mw) of the polymer of Structure 4 in Example 1 was calculated to be approximately 53,000.

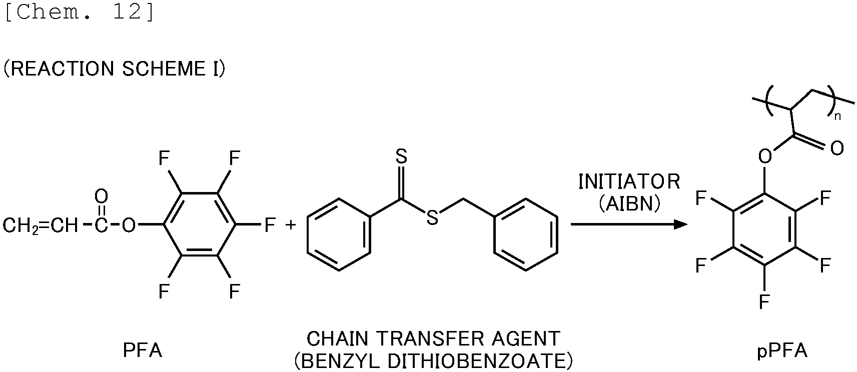

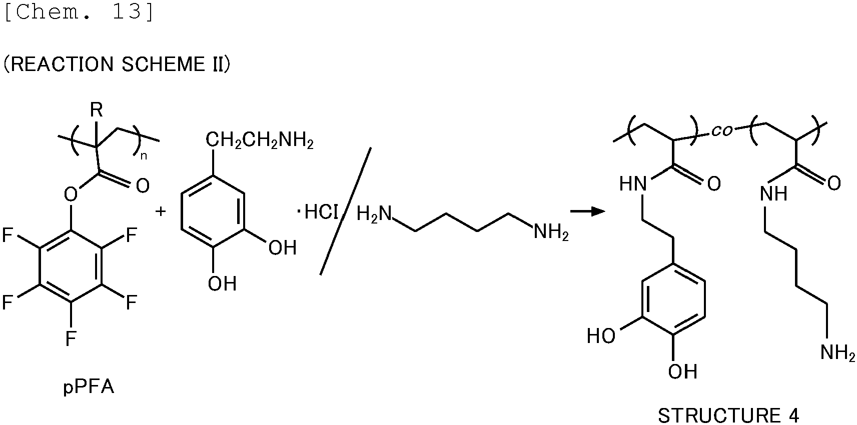

### <Value of ratio (Mw/Mn) of weight-average molecular weight to number-average molecular weight>

Since it was considered that a molecular weight distribution would not significantly change before and after a side chain reaction (Reaction Scheme II in the example of Example 1), an average molecular weight of the polymer before a side chain reaction (pPFA in the example of Example 1), which had high solubility in a solvent used for gel permeation chromatography (GPC), was measured. More specifically, the polymer before the side chain reaction and tetrahydrofuran (THF) were stirred for 10 minutes to yield a solution, and the resultant solution was filtered with a 0.2 µm filter, followed by measuring a weight-average molecular weight (Mw) and a number-average molecular weight (Mn). Based on the values of the weight-average molecular weight (Mw) and the number-molecular weight (Mn), a molecular weight distribution (Mw/Mn) was calculated. THF was used as a development solvent of GPC. During the measurement, the column temperature was 40°C, and the flow rate was 1.0 mL/min.

### <Viscosity of undiluted solution>

Among the constituent components of the dental adhesive composition (Table 2), polymerizable monomers were blended with Component A in each example. The formulation was performed at a weight ratio of polymer : HEMA : TEGDMA : Bis-GMA of 1 : 1 : 2 : 3.4. The formulated solution was stirred by a stirrer for 1 hour to prepare an undiluted solution for measuring a viscosity. In measurement of the viscosity, after conditioning a B-type viscometer (Toki Sangyo Co., Ltd.) to be at a constant temperature of 23°C, the undiluted solution was collected by 0.6 mL, and the collected solution was left to stand for 5 minutes to condition the solution to be at a constant temperature. The measurement was performed for 5 minutes at 10 rpm, and an arithmetic mean of viscosity values obtained in the period from 2 minutes to 5 minutes during the measurement was determined as a viscosity of the undiluted solution. The smaller the viscosity of the undiluted solution is, higher and more preferred the dispersibility of Component A in the polymerizable monomer is.

### <Consistency of paste>

The dental adhesive composition prepared in each example was loaded into a polypropylene syringe, followed by centrifugal degassing. Then, the dental adhesive composition in the syringe was left to stand in a thermostat of 23°C for 24 hours, to thereby prepare a composition sample for a consistency test. The composition sample was collected by 0.1 g, and was left to stand on a polyester film (5 cm × 5 cm) inside a thermostat having temperature of 23°C and humidity of 50 RH% in a manner such that the composition sample was piled up at a center of the polyester film. Another polyester film (5 cm × 5 cm) was placed to over the composition sample, and a glass plate and a weight were sequentially placed thereon. Ten seconds after applying a load of 860 g in total, a major axis and a minor axis of the sample were measured, and an arithmetic mean of the both values was determined as a consistency (mm). Note that, the major axis of the sample means the largest diameter among diameters passing through the center of the sample, and the minor axis of the sample means the diameter orthogonal to the major axis of the sample among the diameters passing through the center of the sample. The evaluation criteria of the paste consistency was as follows:
A: 23 mm or greater
B: 17 mm or greater and less than 23 mm
C: less 17 mm

### <Shear bond strength (to enamel)>

A lip side of a surface of an incisor of a bottom jaw of a cattle was polished with #120 silicon carbide paper (manufactured by Marumoto Struers) under a flow of water to expose a flat surface of enamel, thereby obtaining a bonding target sample. The obtained bonding target sample was further polished with #400 silicon carbide paper (manufactured by Marumoto Struers) under a flow of water to prepare a smooth surface. After completing the polishing, water on the surface of the bonding target sample was dried by air blowing. As a pretreatment, a small amount of the dental adhesive composition prepared in each example was applied on the dried smooth surface of the bonding target sample, followed by agitating for 10 seconds with an applicator. A plate-like mold (manufactured by ULTRADENT), in which a through-hole for material filing was formed at a center, was fixed to the sample. The through-hole had a diameter of 2.38 mm and a depth of 2.67 mm. Then, the smooth surface of the bonding target sample on which the dental adhesive composition had been applied was irradiated with light for 10 seconds over the mold via the through-hole of the mold using a dental polymerization visible-ray irradiator (product name: G-Light Prima II Plus, manufactured by GC). Subsequently, a recess formed by the smooth surface of the bonding target sample and the through-hole was filled with the same dental adhesive composition as the above dental adhesive composition used in the small amount, and the dental adhesive composition was irradiated with light for 10 seconds over the mold using the dental polymerization visible-ray irradiator in the same manner as above to cure the dental adhesive composition, thereby obtaining a cured product. Then, the mold was removed to prepare a test piece. As the test piece, 5 test pieces were produced for each example, and were left to stand in a thermostat of 37°C for 24 hours. Thereafter, the test pieces were immersed in water of 37°C for 24 hours, followed by measuring a shear bond strength of each of the 5 test pieces by a precision universal tester (product name: Autograph, manufactured by Shimadzu Corporation) at a crosshead speed of **1.0** mm/min, and an arithmetic mean of the measured values was determined. The evaluation criteria of the shear bond strength enamel was as follows:
A: 15 MPa or greater
B: 10 MPa or greater and less than 15 MPa
C: less than 10 MPa

Within the above evaluation criteria, 10 MPa or greater (A and B) is a suitable bond strength as a dental composite resin.

### <Shear bond strength (to dentin)>

A shear bond strength was measured and an average value thereof was determined in the same manner as above, except that a bonding target sample obtained by exposing a flat surface of dentin was used. The evaluation criteria of the shear bond strength to dentin was as follows:
A: 10 MPa or greater
B: 5 MPa or greater and less than 10 MPa
C: less than 5 MPa

Within the above evaluation criteria, 5 MPa or greater (A and B) is a suitable bond strength as a dental composite resin.

### <Irritation>

To 1 g of the composition of each example, 0.5 mL of ethanol and 0.5 mL of water were added. The resultant mixture was stirred for 10 minutes, thereby obtaining a solution. After performing a 3-point calibration, pH of each solution was measured by a pH meter. The irritation was evaluated based on the following criteria:
A: pH 3 or higher and pH 10 or lower
B: pH 2 or higher and lower than pH 3
C: lower than pH 2 or higher than pH 10

When pH is 3 or higher and 10 or lower, the dental adhesive composition is unlikely to cause irritation to an oral cavity, and desired usability can be achieved.

The results of Examples and Comparative Examples demonstrated that the dental adhesive compositions (Examples 1 to 9) each including the specific polymer having a constituent unit of any of above Structures 1 to 4 and having the weight-average molecular weight (Mw) of 2,000 or greater and 55,000 or less exhibited desirable results in all of the viscosity of the undiluted solution, the paste consistency, the bond strength to the dental tissues, and irritation. Particularly, the dental adhesive compositions (Examples 4 to 7) each including the polymer having the weight-average molecular weight (Mw) of 5,000 or greater and 15,000 or less had excellent bond strength to both enamel and dentin.

Conversely, in the case of the polymer including the constituent unit of any of above Structures 1 to 4, but having the weight-average molecular weight (Mw) outside the range of 2,000 or greater and 55,000 or less, the dispersibility of the polymer was not sufficient so that it was difficult to blend the polymer with other components (Comparative Examples 4 and 5), and even if the polymer could be blended with other components, the desirable results could not be obtained in the viscosity of the undiluted solution, the paste consistency, and the bond strength to dental tissues (Comparative Example 1). Moreover, the dental adhesive compositions (Comparative Examples 2 and 3) each including, instead of the polymer, the monomer that could be a constituent component of a polymer had the insufficient bond strength. Moreover, the composition (Comparative Example 6), in which MDP was blended without blending the above specific polymer, the desirable result was not obtained in the evaluation of irritation.

While embodiments of the present invention have been described above, the present invention is not limited to specific embodiments, and various modifications and variations are possible within the scope of the invention as claimed.

The present application is based on and claims priority to Japanese Patent Application No. 2022-061107, filed March 31, 2022, the contents of which are incorporated herein by reference.

## Claims

1. A dental adhesive composition, comprising:
a polymer including at least a constituent unit represented by a formula (1), and having a weight-average molecular weight (Mw) of 2,000 or greater and 55,000 or less, 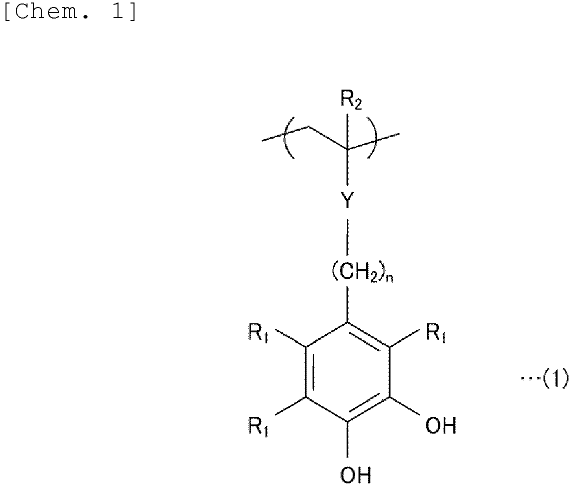 [in the formula (1), Y is an amide bond or an ester bond, n is an integer of 1 or greater and 10 or less, multiple R₁ are each independently a hydrogen atom, a hydroxyl group, or an alkyl group having a carbon number of 1 or greater and 6 or less, and R₂ is a hydrogen atom or an alkyl group having a carbon number of 1 or greater and 6 or less].

2. The dental adhesive composition according to claim 1,
wherein the polymer is a random copolymer including the constituent unit represented by the formula (1) and a constituent unit represented by a formula (2), 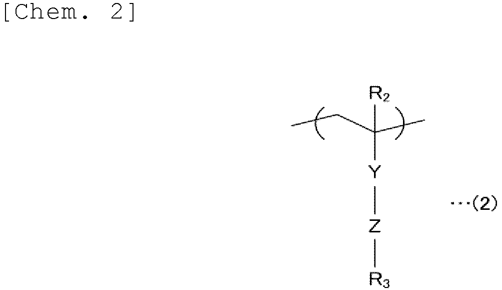 [in the formula (2), Y is an amide bond or an ester bond, Z is an alkylene group having a carbon number of 1 or greater and 10 or less or ethylene glycol having 1 or more repeating units and 5 or less repeating units, R₂ is a hydrogen atom or an alkyl group having a carbon number of 1 or greater and 6 or less, and R₃ is a hydrogen atom, a methyl group, or an amino group].

3. The dental adhesive composition according to claim 1 or 2,
wherein a value of a ratio (Mw/Mn) of a weight-average molecular weight of the polymer to a number-average molecular weight of the polymer is 1 or greater and 2 or less.

4. The dental adhesive composition according to any one of claims 1 to 3,
wherein the constituent unit represented by the formula (1) includes a catechol group.

5. The dental adhesive composition according to any one of claims 1 to 4, further comprising:
a polymerizable monomer,
wherein an amount of the polymer is 5 parts by mass or greater and 300 parts by mass or less relative to 100 parts by mass of the polymerizable monomer.

6. The dental adhesive composition according to any one of claims 1 to 5,
wherein an amount of 10-methacryloyloxydecyl dihydrogen phosphate is 0% by mass, or greater than 0% by mass and 10% by mass or less relative to a total amount of the dental adhesive composition.
